# EUROPEAN PATENT APPLICATION

(11) **EP 1 884 522 A1**
(43) Date of publication of application: **06.02.2008**
(21) Application number: 06118163.2
(22) Date of filing: 31.07.2006
(51) Int. Cl.: C07K 16/18, G01N 33/573, A61P 35/00

(54) **Antibody binding to a polo-like kinase 1 phosphorylation site of the human cohesin Wapl**

(71) Applicant: Boehringer Ingelheim International GmbH, 55216 Ingelheim am Rhein (DE)
(72) Inventor: Hegemann, Bjoern, 1020 Wien (AT); Peters, Jan-Michael, 2103 Langenzersdorf (AT); Hudecz, Otto, 3400 Klosterneuburg (AT); Mechtler, Karl, 2126 Ladendorf (AT)
(74) Representative: Hammann, Heinz

(57) **Abstract**

Antibodies recognizing Plkl-dependent phosphorylation of the cohesion subunit Wap1 and methods for using such antibodies for determining Plk1 activity, e.g. in clinical trials for assessing the therapeutic effect of Plkl inhibitors.

## Description

The present invention relates to the therapy of cancer with inhibitors of Polo-like kinase 1 (P1k1), in particular to methods for determining the activity of P1k1. in human cells.

The cell cycle has been recognized as a major target of therapeutic intervention in the treatment of cancer. Therefore, inhibitors that interfere with proteins of the cell cycle have gained increasing importance as anti-cancer drugs.

Anti-cancer drugs are sometimes judged for their effect on a surrogate marker (also termed "biomarker") that may predict clinical benefit to the patient, such as regression of tumors. When monitoring a patient's response to a cell cycle inhibitor, a surrogate marker is the basis for clear demonstration that an administered compound and a chosen dose and treatment schedule have reached levels sufficient to inhibit the activity of the cell cycle target.

However, traditional clinical end points have proven difficult to apply in the evaluation of molecularly targeted therapies; standard clinical trial endpoints that are used for cytotoxic compounds have been found to be insufficient for the evaluation of molecularly targeted, e.g. cell cycle-targeted, antiproliferative agents.

In the development of molecularly targeted, e.g. cell cycle-targeted, inhibitors, it is important to have endpoint assessments available for (i) the efficacy of the compound in modulating the activity of its molecular target, and (ii) the relationship between target modulation and clinical response.

Thus, the availability of biomarkers (i.e. a measurable biological activity that indirectly indicates the effect of treatment on disease state) is one of the key prerequisites for the development of minimally invasive techniques to assess an inhibitor's efficacy and, consequently, both for the clinical evaluation of the drug and, in the long run, for monitoring the efficacy of the approved drug in therapy.

The molecularly targeted agent STI-571 (the Novartis compound also known as Gleevec) is a positive example for the contribution of a surrogate marker to successful clinical development: STI-571 was developed as a tyrosine kinase inhibitor with specificity for the Abl tyrosine kinase, although c-Kit and the platelet-derived growth factor receptor are also targets. In chronic myelogenous leukemia, 95% of patients have the Philadelphia chromosome that results in generation of the oncogenic Bcr-Abl fusion protein. Studies have shown that the activity of this oncogenic kinase is sufficient to cause the disease, thus allowing therapeutic efforts to be focused on a highly appropriate patient population (Druker and Lydon, 2000). Inhibition of tyrosine kinase activity was readily assayed by the analysis of levels of phospho-CrkL, the predominant Bcr-Abl substrate in neutrophils. This endpoint assay assisted in the identification of a biologically active dose. The rapid and successful clinical development of Gleevec largely rests on the fact that the disease in the patient population tested was dependent on ABL activity, and there were methods available for the determination of ABL inhibition and disease remission in patients.

Another example of a cell cycle target whose activity can be readily measured is CDK4; this assay employs detection of pRB phosphorylation by CDK4 (Fry, et al., 2001). Western blot analysis of lysates prepared from compound-treated cells were performed using an antibody that detected phosphorylation of pRb at serine 780, a site that is thought to be phosphorylated by CDK4.

There are many examples of substrate phosphorylation sites that can serve as useful readouts for cellular inhibition of a particular molecular target. With reference to the cell cycle in particular, phosphorylation on serine 216 of the dual specificity phosphatase Cdc25C has been used to monitor the cellular activity of the Chk1 cell cycle checkpoint kinase (Graves, et al., 2000). Similarly, phosphorylation of the core histone H3 on serine 10 can be used to monitor the efficacy of inhibitors of the kinase Aurora B (Ditchfield et al., 2003; Hauf et al., 2003).

Furthermore, Wee1 activity has been examined by detection of phosphorylation on tyrosine 15 and threonine 14 on CDK1 using phosphospecific antibodies to these sites (Wang, et al., 2001).

Importantly, total substrate levels as well as the level of phosphorylation on a particular site should always be determined to monitor specific signal loss (Fry, et al., 2001).

P1k 1 is another cell cycle kinase that has been considered as a target for anti-cancer therapy. This is due to increasing evidence that P1k1 forms part of the regulatory circuit that controls entry into and progression through mitosis.
P1k1 belongs to the family of serine/threonine kinases, and it has been shown to be required for spindle formation and chromosome segregation during mitosis. The essential role of P1k1 orthologues in controlling the progression of dividing cells through mitosis has been demonstrated by genetic means in several model organisms (e.g., Drosophila melanogaster, Xenopus laevis, yeast) as well as with antibody injection or siRNA knock-down experiments in human tumor cells (Llamazares et al., 1991; Kitada et al., 1993; Lane and Nigg, 1996; Spankuch-Schmitt, et al., 2002; Liu and Erikson, 2002; Liu and Erikson, 2003; Sumara et al., 2004; van Vugt et al., 2004). It has also been shown that P1k1 mRNA expression correlates with the mitotic activity of cells and the prognosis of lung cancer patients (Yuan, et al., 1997). Inhibitors of P1k1 are therefore thought to be promising drugs for the treatment of neoplastic diseases (Strebhardt and Ullrich, 2006). However, to date no specific biomarkers and, consequently, no assays are available to directly monitor the effect of such a drug on the cellular activity of P1k1. Thus, in order to determine the in vivo efficacy of P1k1 inhibitors and to select patients for being treated with P1k1 inhibitors, there is a need for P1k1 biomarkers.

It was therefore an object of the invention to provide reliable biomarkers for assessing P1k1 activity and, in particular, for selecting patients for treatment with a P1k1 inhibitor and for assessing a drug's effect on the activity of P1k1 during therapy.

The solution of the problem underlying the invention is based on the consideration that a desirable biomarker would be one or more protein phosphorylation sites whose generation in vivo depends on P1k1 activity. If such sites can be identified, antibodies can be raised against them and can be used in different immunological assays to determine the presence or absence of P1k1 activity in cells and tissues.

At present, only very few phosphorylation sites are known whose generation is thought to depend on P1k1 (reviewed in Barr et al., 2004), but none of these have been rigorously validated as sites specifically modified by P1k1 in vivo.

The experiments that led to this invention are based on a study of human Wap1, a protein that interacts with the cohesin complex.

After replication of the genome during S-phase of the cell cycle, each chromosome contains two identical DNA molecules, called sister chromatids. These sister chromatids are physically connected to each other until chromosome segregation occurs during anaphase of mitosis or meiosis. This cohesion between sister chromatids is mediated by a protein complex which is called cohesin (reviewed by Nasmyth et al., 2001). In human cells, it has recently been discovered that cohesin is bound to a protein called Wap1, and that Wap1 is required for the dissociation of cohesin from chromosome arms during prophase and prometaphase of mitosis (S. Kueng, B. Hegemann, B. Peters, J.J. Lipp, A. Schleiffer, K. Mechtler and J.-M. Peters, unpublished observations). Cohesin is known to be phosphorylated in mitosis, and this modification is believed to be mediated by P1k1 (Losada et al., 2002; Sumara et al., 2002; Gimenez-Abian et al., 2004; Hauf et al., 2005). Because Wap1 and cohesin physically interact with each other, the inventors tested the possibility that Wap1 may also be a substrate of P1k1 and if yes, if such phosphorylation sites could serve as biomarkers for P1k1 activity in vivo.

To solve the problem underlying the invention, the inventors therefore sought to determine if Wap1 is phosphorylated, and if so,
a) on which amino acid residues Wap1 is phosphorylated,
b) if any of these modifications is generated specifically in mitosis, and
c) if the generation of any such sites depends on P1k1 activity in vivo.

Next, the inventors sought to determine which of the identified phosphorylation sites depend on P1k1 activity in vivo and could thus be useful as biomarkers for P1k1 activity. To address these questions, the inventors developed a method that combines chemical biology, protein affinity purification and mass spectrometry.

This method which was used in the experiments of the invention to determine the P1k1-dependent phosphorylation sites on Wap1, can be used as a generic method to screen for biomarkers of various enzyme activities:
Since protein function is often regulated by post-translational modifications, e.g. phosphorylation, post-translational modifications on a protein can be used as readout for enzyme activity, i.e. as a biomarker. To be able to easily detect the phosphorylation state or another post-translational modification state of a target protein and to identify on which enzyme such modifications depend, the inventors developed the following method.

In a first step of this method, cultured cells, e.g. from a tumor cell line in which the enzyme of interest is active (in the case of the present invention the kinase P1k1), are treated with an inhibitor of the enzyme of interest, or left untreated (control cells). After a period of time that is sufficient for the inhibitor to exert its effect on the activity of the enzyme of interest, the target protein is isolated from both the inhibitor-treated and untreated cells by affinity purification and analysed by mass spectrometry under conditions suitable for identifying the post-translational modifications on the target protein. Post-translational modifications that are present on the untreated cells and that are absent on the inhibitor-treated cells are those that are dependent on the enzyme of interest and can thus serve as biomarkers.

Antibodies can then be generated against the identified modifications and can be used to monitor the enzyme's activity in cells and tissues.

The described method has wide applications in all relevant biological pathways in which enzymes post-translationally modify target proteins, provided such modifications can be detected by mass spectrometry. Examples for such modifications are: acetylation, alkylation, biotinylation, glycosylation, lipoylation, phosphorylation, sulfation, ubiquitination, sumoylation or proteolytic processing. In the case of the present invention, the novel method was used to detect phosphorylation sites on cohesin subunits that are dependent on the mitotic kinase P1k1 by using the P1k1 inhibitor BI 2536 and a purification and mass spectrometry protocol designed to detect phosphorylation sites on the target proteins of P1k1. To detect phosphorylation sites generated by other kinases of interest, a range of inhibitors can be used (e.g. Hesperadin to inhibit Aurora B kinase (Hauf et al. 2003), Roscovitine to inhibit Cyclin dependent kinase 1 (Meijer L et al. 1997) or Gleevec to inhibit Abl tyrosine kinase.

The same principle can be applied to other post-translational modifications like acetylation using acetylase inhibitors (e.g. trichostatin A (Minucci S et al. 1997)) or methylation using methylase inhibitors (e.g. chaetocin (Greiner D et al. 2005)). Both modifications are especially abundant on histone proteins and can be identified by mass spectrometry methods (Rice JC et al. 2003; Beck HC et al. 2006). Many other post-translational modifications can be detected by various mass spectrometry methods (reviewed in Cantin GT and Yates JR 3rd (2004)). In combination with the vast range of enzyme inhibitors, the method of the invention offers plenty possibilities to detect post-translational modification-dependent biomarkers. The protocol can be easily applied to any given target protein of interest by adapting standard affinity purification protocols to that protein, e.g. by providing the specific antibodies, and combining the purification method with the mass spectrometry method suitable for detecting the post-translational modification of interest.

Using the above-described method, the inventors purified Wap1 from human cells that were in interphase (where P1k1 is not active), and from human cells that had been arrested in mitosis (where P1k1 is active), and from human mitotic cells in which P1k1 activity had been inhibited. To inhibit P1k1 activity, the specific P1k1 inhibitor BI 2536 was used, an ATP-competitive kinase inhibitor derived from a novel chemical series, the dihydropteridinones (WO 03/020722). The structure of BI 2536 is

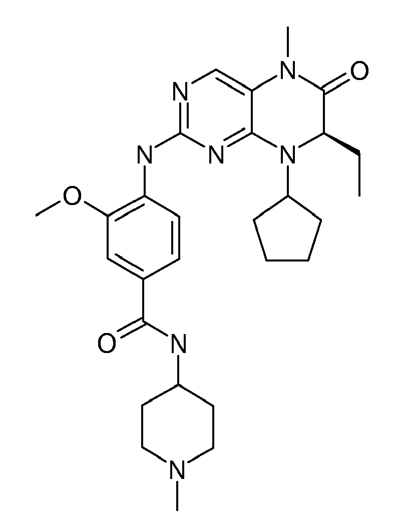

Next, phosphorylation sites were identified in all three Wap1 samples by mass spectrometry. A comparison of the three datasets revealed that Wap1 is phosphorylated on several amino acid residues, that several of these phosphorylation sites are only generated in mitotic cells, and that several of the mitotic sites are missing when Wap1 has been purified from cells which had been treated with BI 2536, indicating that the generation of these latter sites must depend on P1k1 activity in vivo.

Next, the inventors confirmed that the BI 2536 sensitive phosphorylation sites listed above can be used as biomarkers for P1k1 activity. To test this, antibodies were raised against a phosphorylated peptide that represents a fragment of Wap1 which is phosphorylated on serine 465. Antibodies were purified from the resulting antisera that recognize the Wap1 peptide when it is phosphorylated on serine 465, but these antibodies do not react with an otherwise identical Wap1 peptide that is not phosphorylated on this amino acid residue. The inventors showed that these antibodies react with Wap1 in immunoblot experiments only when Wap1 is isolated from mitotic cells where Wap1 is phoshorylated on serine 465. In contrast, the antibodies do not react with Wap1 when the protein is isolated from interphase cells where Wap1 is not phosphorylated on serine 465, or with mitotic Wap1 that has been treated with a protein phosphatase in vitro. The antibodies do also not recognize Wap1 isolated from mitotic cells when these have been pre-treated with the P1k1 inhibitor BI 2536. On the other hand, these antibodies can also react with Wap1 purified from interphase cells if the protein has been incubated in vitro with P1k1 and ATP. Together, these observations indicate that the antibodies react with Wap1 only when Wap1 has been phosphorylated by P1k1.

Further immunoblot experiments revealed that the antibodies raised against phosphorylated Wap1 also react with other unidentified proteins in total protein lysates from HeLa cells. These cross-reactions were predominantly observed with proteins from mitotic cells and were reduced when extracts from interphase cells were analyzed, and these cross-reactions were also reduced when extracts were prepared from mitotic cells that had been pre-treated with BI 2536. The antibodies raised against phosphorylated Wap1 are therefore not only reacting with Wap1, but also with other yet unidentified P1k1 substrates. From the obtained results it can be concluded that the observed reactions of the antibodies with yet unidentified antigens partially depend on mitotic phosphorylation mediated by P1k1.

To test if anti-Wap1 antibodies of the present invention can be used to determine P1k1 activity in vivo, the reactivity of the antibodies with human cultured cells was analyzed in immunofluorescence microscopy experiments. The inventors observed that the antibodies did stain mitotic cells, but did not react with cells in interphase, consistent with the possibility that the antibodies react only with the mitotically phosphorylated proteins. Importantly, the inventors showed that the reactivity of the phospho-antibodies with human mitotic cells was abolished when these cells had been treated with the P1k1 inhibitor BI 2536. This result indicates that the phospho-antibodies originally raised against Wap1 can be used as biomarkers for P1k1 activity. This notion was further supported by experiments which showed that the reactivity of the phospho-antibodies with human cells is also decreased when the levels of P1k1 had been reduced in these cells by RNAi. This observation indicates that BI 2536 inhibits the reactivity of the phospho-antibodies by inactivating P1k1, and not by inhibition of another unknown enzyme.

It is presently unknown which proteins in human cells are recognized by the anti-Wap1 phospho-antibodies in immunofluorescence microscopy experiments. The immunoblot experiments indicate that Wap1 is one of the proteins that is recognized by these antibodies but by no means the only one. However, it is important to note that these phospho-antibodies can nevertheless be used as biomarkers for P1k1 activity in vivo since the reactivity of the antibodies is sensitive to treatment with BI 2536.

There are numerous examples of previously developed antibodies that have been used successfully as biomarkers, although their antigens were initially unknown or are still unknown today. In the area of cell cycle and cancer research, there are several examples for such "orphan" antibodies. The monoclonal antibody Ki67 reacts specifically with proliferating cells and is therefore widely used in oncology as a routine marker to determine the proliferation index of tumor and control tissues (Nawa et al., 1996). The Ki67 antibody was already used for this application for many years before its antigen was finally identified (Naiem et al, 1983; Schluter, 1993). Other examples are the MPM2 and 3F3/2 monoclonal antibodies (Davis, 1983; Westendorf, 1994; Cyert, 1988). Both of these recognize a variety of different mitotically phosphorylated proteins, but the identity of most of these proteins has to date remained unknown. Both antibodies have nevertheless be used as biomarkers in studies of mitotic mechanisms.

The study carried out by the inventors provides proof of principle that the strategy described above can be used to develop biomarker assays for P1k1 activity.

Phospho-specific antibodies can be raised, as described in the Examples for serine 465, against the other BI 2536 sensitive phosphorylation sites that have been identified on Wap1. Furthermore, the existing anti-Wap1 phospho-antibodies can be used to identify their antigens by immunoprecipitation and mass spectrometry as follows: Anti-Wap1 phospho-antibodies can be incubated with low speed supernatants of cell extracts from mitotic cells and with low speed supernatants of cell extracts from mitotic cells treated with a P1k1 inhibitor, e.g. BI 2536. Proteins binding to the antibodies can then be eluted and analysed by mass spectrometry. The protein or proteins that are detected in eluates from mitotic cells but not in eluates from mitotic cells treated with BI2536 represent the P1k1-dependent antigens recognised by anti-Wap1 phospho-antibodies. Antibodies against these antigens can be generated to analyse their mitotic P1k1- dependent phosphorylation sites using the chemical biology / mass spectrometry method described above. These P1k1-dependent phosphorylation sites can then be used as additional P1k1 biomarkers.

Hence, based on the identification of phosphorylation sites of critical P1k1 substrates, antibodies can be provided that bind specifically to the identified phosphorylation sites and that are useful in assays for assessing the phosphorylation of Wap1 and/or other yet to be identified P1k1 substrates, said phosphorylation being indicative of P1k1 activity.

In the experiment of the invention, eight phosphorylation sites in total were detected on Wap1 (SEQ ID NO:1). Of these phosphorylation sites, the phosphorylation sites at serine 262, serine 308 or serine 311, serine 465, serine 528, serine 629 or serine 634 or serine 636, serine 988 and at serine 1154 are detected mitotic cells. The phosphorylation sites at serine 262, serine 308 or serine 311, serine 433 or 439 or serine 442 are also phosphorylated in S-phase cells. Further, the phosphorylation site at serine 629 or serine 634 or serine 636 is also phosphorylated in interphase.

In the experiments with the P1k1 inhibitor BI 2536, it was surprisingly found that all mitosis-specific phosphosites (at serine 465, serine 528, serine 988 and at serine 1154) are sensitive to a specific P1k1 inhibitor, which means that these phosphosites are P1k1-dependent and therefore specific biomarkers for P1k1.

In a first aspect, the present invention provides antibodies which are specifically immunoreactive with a phosphorylated form of Wap1 (SEQ ID NO: 1).

According to this aspect, the present invention relates to an antibody that binds to a phosphorylation site of the human cohesin subunit Wap1 (SEQ ID NO:1) when said phosphorylation site of Wap1 is phosphorylated by P1k1 and that does not bind to said potential phosphorylation site when said phosphorylation site is not phosphorylated.

In a preferred embodiment, said antibody binds to
i) one or more P1k1-dependent phosphorylations at serine 465, serine 528, serine 988 or serine 1154 of Wap1 (SEQ ID NO: 1) and/or
ii) one or more P1k1-dependent phosphorylations on another P1k1 substrate, wherein said phosphorylations are recognized by an antibody generated against a phosphorylation sites of Wap1 defined in i).

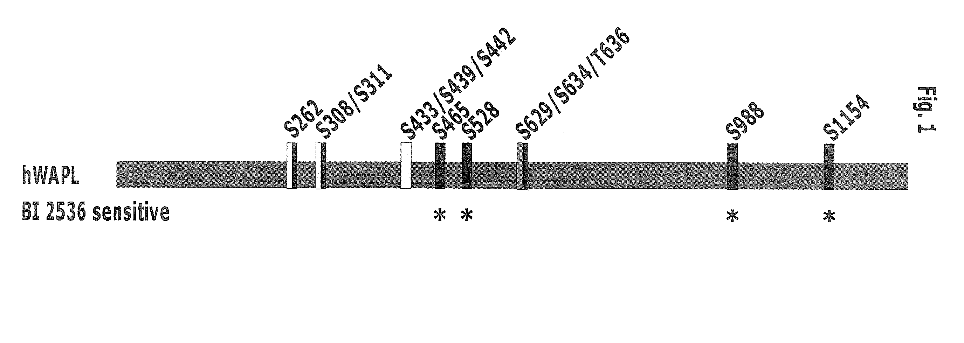

The antibody recognizes a phosphorylation site that is specific for mitotic cells and dependent on P1k1 activity. These phosphorylation sites are apparent from Figure 1.

The term "antibodies" as used herein, refers to immunoglobulin molecules and immunologically active portions of immunoglobulin molecules, i.e., molecules that contain an antigen binding site or paratope. Examples of such antibody molecules are intact immunoglobulin molecules, substantially intact immunoglobulin molecules and those portions of an immunoglobulin molecule that contain the paratope, including those portions known in the art as Fab, Fab', F(ab')₂ and F(v).

The term "other P1k1 substrates" refers to yet unidentified P1k1 substrates whose one or more P1k1-dependent phosphorylations are recognized by an antibody raised against the P1k1-dependent phospho sites of Wap1.

The antibody of the invention may be a polyclonal or monoclonal antibody, preferably, it is monoclonal.

As stated above, an antibody of the invention immunoreacts with an epitopic site of phosphorylated Wap1 and other yet unidentified P1k1 substrates. The term "epitopic site" refers to an antigenic determinant that consists of chemically active surface groupings of molecules such as amino acids or sugar side chains that usually have specific three dimensional structural characteristics, as well as specific charge characteristics. The preferred peptides containing an ectopic site and thus used for immunization to obtain the antibodies of the invention are peptide fragments of Wap1 or other P1k1 substrates consisting essentially of about 9 to 21, preferably about 11 to about 17 amino acid residues, wherein about 4 to 10, preferably about 5 to about 8 amino acid residues are positioned on each side of the phosphorylation site.

For generation of the antibodies of the invention, standard procedures for generating antibodies, in particular phospho-specific antibodies, can be used (Wisdom, 1994).

To obtain phospho-specific antibodies against Wap1 and/or other P1k1 substrates, a series of epitopic peptide fragments of Wap1 and/or other P1k1 substrates containing the respective phosphorylation sites, with a minimum length of six amino acids, usually about 9 to 21, preferably of about 11 to 17 amino acids, are synthesized.

The peptides may be synthesized such that they have a cysteine attached at the C-terminus to permit the unidirectional attachment to a carrier protein through a connecting bridge. Standard methods of conjugation also include the glutaraldehyde method which couples primarily through the α-amino group and ε-amino group, and the Maleimidobenzoyl-N-hydroxysuccinimide ester (MBS) method which couples through sulfhydryl groups. The carrier proteins most commonly used are Keyhole Limpet Hemocyanin (KLH), Bovine Serum Albumin (BSA), Rabbit Serum Albumin (RSA), Ovalbumin or Thyroglobulin (THY).

Synthesis of the phosphorylated peptides and, for specificity control and for affinity purification in the case of polyclonal antibodies, of unphosphorylated peptides, can be done according to standard procedures as described by Wisdom, 1994, e.g. by the standard Merrifield (Boc) solid phase synthesis. Methods suitable for synthesizing the phospho-peptides for immunization and of the ultimately resulting antibodies of the invention are also described by Wisdom, 1994, and inter alia, for serine-phosphorylated antibodies by Lewis at al., 1994, and for tyrosine-phosphorylated antibodies in US 5,599,681.

Another method suitable for generating the antibodies of the invention is described in US 6,309,863, which suggests a method for producing polyclonal or monoclonal phospho-specific antibodies by using for immunization a peptide containing a phosphopeptide mimetic.

The invention also provides an immunogenic composition comprising an antigenic phospho-peptide derived from a polypeptide selected from SEQ ID NO:1 as described above, wherein said phospho-peptide.

For generating polyclonal antibodies, animals, usually rabbits, e.g. New Zealand White rabbits, are immunized with the synthetic phosphopeptide (conjugate). IgG from best responding animals, as determined by enzyme-linked immunosorbent assay against the phosphopeptide and corresponding unphosphorylated peptide, is purified by standard methods, e.g. as described in Rena et al., 1999, for the generation of generation of phosphospecific antibodies for FKHR (Forkhead transcription factors FOXO1). In brief, by this method antibodies reactive with the unphosphorylated peptide are removed by adsorption using an affinity column loaded with the corresponding unphosphorylated peptide, followed by affinity chromatography, e.g. on CH-Sepharose to which the corresponding phosphopeptide antigen had been covalently attached, and elution of the phosphospecific antibodies.

Preferably, the antibodies of the invention are monoclonal antibodies that bind specifically to the predetermined phosphoprotein epitope. Monoclonal antibody secreting hybridomas are produced using the phospho-peptides in conventional techniques (see e.g., Harlow and Lane, 1988). Phospho-specific monoclonal antibodies are usually produced by murine hybridomas formed by fusion of:
a) a mouse myeloma or hybridoma which does not secrete antibody with,
b) a murine spleen cell which secretes antibodies, obtained from a mouse immunized with a mimetic-containing polypeptide antigen.

Typically, several mice are immunized with a primary injection of antigen followed by a number of boosting injections. During or after the immunization procedure, sera from the mice are screened to identify mice which have mounted a substantial immune response. For selected mice, the spleen cells are obtained and fusions are performed. Suitable fusion techniques include, for example, the Sendai virus technique (Kohler and Milstein, 1975), or the polyethylene glycol method (Kennet, 1980).

The resulting hybridomas are then screened for production of antibodies specific for the antigen. Suitable screening technique are a solid phase radioimmunoassay or ELISA techniques, e.g. coating 96-well plates with phospho- or nonphosphopeptides, incubating with hybridoma supernatant and then using a labeled secondary antibody, e.g. a horseradish peroxidase-conjugated antibody, for detection. Using these technique, a solid phase is prepared by coupling the appropriate antigen to an insoluble matrix. The immunoadsorbent is brought into contact with culture supernatants of hybridomas. After a period of incubation, the solid phase is separated from the supernatants, then contacted with a labeled antibody against murine immunoglobulin. Label associated with the immunoadsorbent indicates the presence of hybridoma products reactive to the antigen.

According to traditional methods, the monoclonal antibodies are produced in large quantities by injecting antibody producing hybridomas into the peritoneal cavity of mice and after an appropriate time, harvesting ascitic fluid from the mice. The thus obtained monoclonal antibodies are then isolated from the fluid.

Preferably, alternative methods are used by which the hybridomas are cultured *in vitro;* such methods have been recently developed, mainly due to animal-welfare issues:
The simplest approach for producing monoclonal antibodies *in vitro* is to grow the hybridoma cultures in batches and purify the monoclonal antibodies from the culture medium. Serum-free media specifically formulated to support the growth of hybridoma cell lines are commercially available. After adaptation of the cells to serum-free media, cell cultures are allowed to incubate in commonly used tissue-culture flasks under standard growth conditions; the monoclonal antibody is then harvested from the medium.

To increase cell viability and the density at which the cells grow and thus increase monoclonal antibody concentration, methods can be used that employ spinner flasks or roller bottles, alternatively, commercially available gas-permeable bags may be used. To permit cells to grow at high densities, the use of a barrier, either a hollow fiber or a membrane, with a low-molecular-weight cutoff has been implemented in several devices for in *vitro culture* of hybridoma. In these semipermeable-membrane-based systems, the cells are isolated and the monoclonal antibodies produced in a small chamber separated by a barrier from a larger compartment that contains the culture media. This compartmentalization makes it possible to achieve monoclonal antibody concentrations comparable with those in mouse ascites. In a hollow-fiber bioreactor, the medium is continuously pumped through a circuit that consists of a hollow-fiber cartridge, gas-permeable tubing that oxygenates the media, and a medium reservoir. The hollow-fiber cartridge is composed of semipermeable multiple fibers that run through a chamber that contains hybridoma cells growing at high density. Such hollow-fiber bioreactors are commercially available, e.g. the so-called "Tecnomouse" from Integra Biosciences).

Next, the resulting antibodies are, using standard technology (e.g. Wisdom, 1994), purified and, in order to determine the extent of their phosphospecificity, characterized by immunoassays, e.g. enzyme-linked immunosorbent assay (ELISA), for binding to the respective phospho- and non-phosphopeptides. To this end phosphorylated and non-phosphorylated peptides are coated onto 96-well microtiter plates and, after blocking unspecific binding sites with excess protein, incubated with the solution containing the antibodies to be characterized (e.g. hybridoma supernatant, dilutions of purified antibodies). After allowing the antibodies to bind, unbound antibodies are washed off and a secondary antibody directed towards either mouse IgG or rabbit IgG is added to the wells. The secondary antibody is typically conjugated with an enzyme such as horse radish peroxidase (HRP) in order to visualize the specific binding of primary antibody to the coated peptide by the addition of a suitable substrate (e.g. 2,2'-Azino-bis(3-ethylbenzothiazoline-6-sulfonic acid) diammonium salt from Sigma Aldrich)

Antibodies specific for a given phospho-epitope should recognize the phosphorylated but not the non-phosphorylated form of the peptide used. Subsequently, the antibodies of the invention are analysed by standard Western blotting technique to further determine specificity against Wap1 and/or other P1k1 substrates, respectively. To this end, whole cell lysates from human tumor cell lines are separated by SDS-PAGE and blotted onto e.g. nitrocellulose membranes. The membrane is subsequently incubated with blocking solution and then the antibody directed against the phosphoepitope(s) is added. After incubation, unbound antibody is washed away and an secondary antibody conjugated e.g. with HRP (horse raddish peroxidase) is added. After washing off unbound antibodies the bound antibodies are visualized by ECL (enhanced chemiluminesce). The antibodies should give a single band at the molecular weight corresponding to Wap1 or the other P1k1 substrates. To check the specificity of the antibodies, incubation of the membrane with the primary (e.g. phosphospecific) antibody is also done in the presence of phospho- or nonphospho- polypeptide selected from SEQ ID NO:1 or the sequence encoding the P1k1 substrate, as described above. The obtained signal is considered to be specific if it can be competed away with the phospho-polypeptide but not with the corresponding non-phospho-polypeptide.

In the case that there are sites that have not been unambiguously identified as phospho-sites, two antibodies are raised against peptides which are each phosphorylated on only one of the sites in question. The obtained antibodies, each one immunoreactive against only one of the phosphorylation sites in question, are tested for reactivity on the P1k1 substrate in its phosphorylated state (e.g. isolated from mitotic cells). The site that is recognized by one antibody (or possibly both sites are recognised each by one antibody in the case that both sites are P1k1-dependent phospho-sites) can be confirmed as the relevant P1k1 biomarker. In the case that both sites are P1k1-dependent, a monoclonal antibody can be generated that recognizes both sites.

As shown in the experiments of the invention, antibodies can be selected that distinguish between mitotically arrested and logarithmically growing cells. To this end, a lysate of mitotically arrested cells (in which P1k1 substrates are predominantly present in their phosphorylated state due to P1k1 activity) is contacted with a candidate antibody and the reactivity of the antibody is determined by Western blot analysis as described above. The obtained signal is compared side by side with the signal obtained with lysates of logarithmically growing cells. Antibodies which yield a strong signal in lysates from mitotically arrested cells but do only yield a very weak signal in lysates from logarithmically growing cells are best suited to be used in assays for assessing P1k1 activity.

The specificity of the antibodies of the invention can be further evaluated by immunohistochemistry in tissue sections from human tumors. For this purpose, xenografts from different tumor types are prepared, the xenografted mice are treated with BI 2536 or another P1k1 inhibitor, using the dose and schedule necessary to achieve a tumor regression. The mice are sacrificed, the tumors excised and processed for immunohistochemical analysis.

In addition, clinical samples from human tumors are analyzed in a similar manner, using the assay conditions established on the xenograft studies: Frozen and paraffin-embedded sections are prepared and analysed in an immunofluorescence assay using the antibodies of the invention, e.g. using an indirect immunofluorescence assay and the avidin-biotin (ABC) immunoperoxidase method described e.g. by. Garin-Chesa et al. 1990. To confirm the specific binding of the antibodies to mitotic cells, double labeling experiments with the mitosis marker phospho-histone-H3 antibody (Upstate) are carried out.

In a further aspect, the invention also provides a method for detecting the presence Wap1 and/or other P1k1 substrates that are phosphorylated in a P1k1-dependent manner, wherein a biological sample suspected of P1k1 activity (and thus containing Wap1 and/or other P1k1 substrates phosphorylated at the relevant sites) is contacted with a phospho-specific antibody of the invention and binding of the antibody is determined. Due to the antibody's ability to distinguish between phosphorylated and non-phosphorylated Wap1 or other P1k1 substrates, the readout of said measurement can be directly correlated with P1k1 activity. In an embodiment of the invention, in order to determine the pharmacodynamics of a drug that acts as a P1k1 inhibitor, i.e. the action of the drug in the body over a period of time, including the processes of absorption, distribution, localisation in the tissues, biotransformation, and excretion, the activity of P1k1 is determined in a biological sample. For determining the pharmacodynamic activity of a drug that acts as a P1k1 inhibitor, the biological sample is derived from a patient who is being treated with said P1k1 inhibitor.

The sample may be any tissue or body fluid from a subject. Ideally, the sample comprises proliferating cells that are positive for cohesin, e.g. tumor cells or, alternatively, cells derived from tissue with mitotic activity, e. g. hair root cells (radix pili cells) or mouth mucosa cells.

In a further aspect, the invention relates to a method for determining a compound's effect on P1k1 activity in an individual treated with a compound by determining *ex vivo* before and after administration of said compound to said individual the presence of phosphorylated Wap1 and/or other P1k1 substrates in a sample and comparing the levels of phosphorylated Wap1 and/or other P1k1 substrates before and after administration of the compound, wherein a reduced level of phosphorylated Wap1 and/or other P1k1 substrates upon administration of the compound is indicative of its inhibitory action on P1k1.

In a variation of this method, the compound's effect on P1k1 activity may be determined over a period of time at pre-defined time points by comparing the levels of phosphorylated Wap1 and/or other P1k1 substrates over said period of time, wherein a reduced level of phosphorylated Wap1 and/or other P1k1 substrates is indicative of a compound's therapeutic effect.

In principle, the above described methods can be conducted by using a single antibody, e.g. an anti-Wap1 antibody, however, two or more assays employing different phospho-specific anti-Wap1 and/or antibodies against an other P1k1 substrate may be done in parallel.

In the above described assays, the desired therapeutic effect of a P1k1 inhibitor should manifest itself in a decrease of phosphorylated Wap1/other P1k1 substrate relative to the overall amount of the substrate protein.

The antibodies of the invention are also useful for determining the specificity of a compound's inhibitory effect on P1k1. To this end, the amount of phosphorylated Wap1 and/or other P1k1 substrate is compared with the level of other cell cycle biomarkers, e.g. with the level of cyclinB, phosphorylated histone H3 or MPM2-reactive signal (or other enzymes or markers expressed in mitosis). Administration of a specific P1k1 inhibitor should result in a decrease of the P1k1-specific phosphorylation signal, while the other cell cycle markers should be unaffected by the compound. Thus, upon treatment of a patient with P1k1 inhibitor the biological sample from a tissue or fluid that normally contains proliferating cells should, at a given time point, show high levels of mitotic markers (e.g. cyclin B, MPM-2 or histon H3 phospho-signal, Ki-67, Aurora kinase etc.) and reduced or no levels of P1k1-specific phosphorylation signal. The degree of the observed mitotic arrest and the degree of downregulation of P1k1-specific phosphorylation signal can be used to assess the pharmacodynamic activity of the administered drug. In insights gained from this evaluation can be used to help guiding the adjustment of drug dose and treatment schedule.

The antibodies of the invention may also be used for selecting patients for treatment with P1k1 inhibitors. To this end, tumor samples from cancer patients are tested for P1k1 activity as described above. Since a high level of phosphorylation, at the relevant sites, of Wap1 and/or other P1k1 substrates in the tumors of patients may be a consequence of aberrant P1k1 activity, it may be expected that these patients are likely to benefit from treatment with a P1k1 inhibitor.

In a further aspect, the invention relates to a method for treating a patient or a patient population having a tumor that correlates with P1k1 activity, comprising the steps of
a) determining whether the tumor of said patient contains one or more P1k1-dependent phosphorylations,
b) selecting a patient or patient population whose tumor has one or more P1k1-dependent phosphorylation(s),
c) administering to said patient a therapeutically effective amount of a P1k1 inhibitor.

In a preferred embodiment, step b) comprises determining one or more P1k1-dependent phosphorylations on Wap1 (SEQ ID NO: 1).

Specifically, step b) comprises determining
iii) one or more P1k1-dependent phosphorylations at serine 465, serine 528, serine 988 or at serine 1154 of Wap1 (SEQ ID NO: 1) and/or
iv) one or more P1k1-dependent phosphorylations on another P1k1 substrate, wherein said phosphorylation is recognized by an antibody generated against a phosphorylation site of Wap1 defined in i).

The P1k1 inhibitor administered in step c) may be BI 2536 or any other therapeutically effective dihydropteridinone compound disclosed in WO 03/020722 or WO 04/076454. Also, other therapeutically active compounds described as P1k1 inhibitors, e.g. in WO 06/063806, WO 05/042505, WO 03/093249, WO 04/043936 and WO 05/019193, may be used.

Alternatively, the P1k1 inhibitor may be an anti-P1k1 si-RNA or antisense molecule as described in WO 03/070283.

Since P1k1 is a kinase that has crucial role in regulating multiple steps in mitosis, the method of the invention is useful for multiple cancer types in a broad indication spectrum, i.e. both solid tumors and hematological malignancies. Examples are non-small-cell lung cancer (NSCLC), breast cancer, prostate cancer, pancreatic adenocarcinoma, B-NHL, colon cancer, acute myeloid leukemia, cancer of the head and neck.

In a further aspect, the invention relates to a method of advertising and selling a P1k1 inhibitor for the treatment of a patient or patient population in need of such treatment, wherein such need is defined by the presence in the tumor of said patient or patient population of
i) one or more P1k1-dependent phosphorylations at serine 465, serine 528, serine 988 or at serine 1154 of Wap1 (SEQ ID NO: 1) and/or
ii) one or more P1k1-dependent phosphorylations on another P1k1 substrate, wherein said phosphorylation is recognized by an antibody generated against a phosphorylation site of Wap1 defined in i).

The methods of the invention for determining phosphorylation status can be carried out using standard immunohistochemical methods. To this end the collected patient sample is prepared for immonohistochemical analysis as it has been described previously (e.g by Harlow and Lane, supra). Briefly, the patient sample containing proliferating cells is embedded into an appropriate matrix (e.g. paraffin or TissueTec for cryopreservation) or smears or spreads are prepared from collected cellular material. Sections from the embedded tissues are generated and are subjected to primary and secondary antibody incubations. P1k1-specific phosphorylation signal or proliferation markers are visualized using peroxidase staining or an equivalent technique. In addition, standard tissue staining techniques such as hematoxilin staining can be used for histological analysis (Harlow and Lane, supra). Instead of using the antibodies of present invention for immunohistochemical analysis, the antibodies may be also used for the analysis of the patient sample by Western Blot (as described above) or isolated patient cells can be subjected to FACS analysis. To this end, such isolated cells are fixed, permeabilized and incubated with primary (e.g. Wap1 phosphorylation specific antibodies and antibodies specific for other mitosis markers as described above) and secondary antibodies conjugated with fluorescent tags. The signals for phosphorylation status and the signal for other mitotic markers is then determined by flow cytometry in a fluorescensce activated cell sorter (FACS).

In another aspect, the antibodies of present invention are useful in screening methods for determining or confirming a compound's ability to inhibit P1k1 in a cell.

Such screening methods can be conducted according to known principles, e.g. by adapting the method described in US 6,287,784 that has been suggested for screening kinase inhibitors by determining autophosphorylation of kinase receptors. In an assay for identifying P1k1 inhibitors, by way of example, a first solid phase is coated with a substantially homogeneous population of cells having P1k1 activity, e.g. proliferating cells that are arrested/synchronized in mitosis (e.g. by incubating them with nocodazole), so that the cells adhere to the first solid phase. Following exposure to the candidate compounds for a period of time sufficient for the compound to exert its inhibitory effect, e.g. for 5 minutes to 5 hours, the adherent cells are solubilized, thereby releasing cell lysate. A second solid phase is coated with a capture antibody that binds to Wap1 and/or other P1k1 substrate at a site different from a phosphorylation site. The cell lysate obtained from the first solid support is added to the second solid support containing the adhering capture antibody in order to capture Wap1 or the other P1k1 substrate, respectively. A washing step is then carried out to remove unbound cell lysate, leaving the captured substrate bound to the second solid support. Next, the captured substrate is exposed to a labelled antibody of the invention, which identifies a phosphorylated residue in the substrate. In a final step, binding of the phospho-antibody to the captured substrate is measured. The obtained signal is compared with the signal obtained from control cells without exposure of compound.

### Brief description of figures:

- Figure 1:: Summary of phosphorylation sites detected on Wap1 by mass spectrometry.
- Figure 2:: Western blot of pWap1 showing it detects a mitosis specific, phosphatase sensitive and BI 2536 sensitive signal.
- Figure 3:: Immunofluorescence staining shows that pWap1 detects only mitotic cell stages in a BI 2536 sensitive manner.
- Figure 4:: Depletion of P1k1 by siRNA followed by immunofluorescence staining with pWap1 confirms that the signal is P1k1 dependent.
- Figure 5:: *In vitro* phoshporylation of immunopurified interphase Wap1 by P1k1 shows that pWap1 recognises a phosphorylation site which is generated directly by P1k1 and is also BI 2536 sensitive *in vitro.*

In the following Examples, if not otherwise stated, the following materials and methods are used:

### Cell lines, growth conditions and siRNA experiments

HeLa cells are grown in DMEM supplemented with 10 % FCS, 0.2 mM L-glutamine, 100 U/ml penicillin, and 100 µg/ml streptomycin. Hydroxyurea, Nocodazole and BI 2536 are used at a concentration of 2 mM, 330 nM and 64 nM respectively.

RNAi: siRNA oligo is synthesized by Ambion. Sense sequence of P1k1 siRNA oligos used is from position 889 to 910 of the P1k1 coding sequence (NM 00530). siRNA-transfection is performed as described (Hirota et al. 2004) at a concentration of 100 nM (P1k1).

### Antibodies

Polyclonal antibodies to human Wap1 are raised against the N-terminal peptide from position 132 to 153 of SEQ ID NO: 1 (designated 986) and the C-terminal peptide from position 1138 to 1163 of SEQ ID NO: 1 (designated 987). The antibodies generated by peptide 987 are used for immunopurification, antibodies generated by peptide 986 are used for western blotting. For immunofluorescence the mouse - anti phospho-Ser10 on histone H3 antibodies (H3S10ph) is from Cell Signalling and used according to manufacturers instructions, the goat - anti SA2 antibodies are from Bethyl labs and used according to manufacturers instructions.

### Mass spectrometry

Immunopurified Wap1-cohesin complex is digested in solution with different proteases (trypsin, Glu-C, chymotrypsin) according to Yates et al., 2000. Proteolytic peptides are applied to a precolumn (PepMAP C18, 0.3 x 5 mm, Dionex) and eluted onto an analytical column (PepMAP C18, 75 µm x 150 mm, Dionex). The eluted peptides are introduced via a nanospray ion source interface (Proxeon) into either an ion trap mass spectrometer (Finnigan LTQ) or an ion trap mass spectrometer coupled to a fourier transform ion cyclotron resonance mass detector (FT-ICR). The mass spectrometer cycles through seven scans, one full mass scan followed by six tandem mass scans of the six most intense ions. Sequenced peptides are put onto an exclusion list for one minute. All tandem mass spectra are searched against the human non-redundant protein database by using algorithms included in Bioworks 3.2 (Thermo Finnigan) and MASCOT 2.1 (Matrix

Science). Any phosphopeptide matched by computer searching algorithms is verified manually.

### Immunoprecipitation

Wap1-Cohesin is purified by peptide antibody affinity chromatography using the 987 antibody. 300 to 500 µg of purified antibody are coupled and cross-linked to Affi-prep protein A beads (BioRad) at 1 µg antibody/µl beads. Wap1 is immunoprecipitated from 6 to 10 ml HeLa extract containing approximately 10 µg protein/µl. Extraction buffer for BI 2536 - treated cells additionally contains 64 nM BI 2536.

### In vitro phosphorylation assay

Wap1-cohesin complex is purified by immunoprecipitation with affinity-purified Wap1 987 antibody. In vitro phosphorylation is performed as described (Kraft et al., 2003) using 10 mM ATP and recombinant 6xHis-tagged P1k1. Recombinant human P1k1 is expressed as a 6xHis-tagged fusion protein using a baculoviral expression system and purified by affinity chromatography using Ni-NTA (QIAGEN). Where indicated, reactions are supplemented with 1 µM BI 2536.

### Immunofluorescence

For immunofluorescence, cells are grown on coverslips and treated with 64 nM BI 2536 45 minutes prior to fixation where indicated. Fixation, immunostaining, and image acquisition are performed as described (Waizenegger et al., 2000).

### Example 1

### Identification of Mitosis-Specific Phosphorylation Sites on Human Wap1

The Smc1, Smc3, SA2 and Scc1 subunits of human cohesin are phosphorylated specifically in mitosis (Hauf et al. 2005). Further it is shown that phosphorylation of SA2 and of Scc1 plays an important role in the regulation of cohesin during mitosis. Recently, another cohesin subunit, Wap1, was identified as a cohesin regulator (S. Kueng and JMP, unpublished results). To detect whether Wap1 is also phosphorylated in mitosis, the mitosis-specific phosphorylation sites on human Wap1 are mapped by mass spectrometry.

Wap1 (SEQ ID NO:1, KIAA0261, accession number NM_015045) is immunopurified using the 987 antibodies from HeLa cells in interphase, arrested at the G1/S transition using hydroxyurea (HU) or arrested at mitosis using nocodazole (noc). To identify phosphorylation sites, purified Wap1 is digested with various proteases in solution and analysed by HPLC-ESI-MS/MS. In total, 8 phosphorylation sites are identified of which 4 were mitosis-specific, 2 are detected in HU- and noc-arrested cells, 1 only in HU-arrested cells and 1 in mitosis and interphase (figure 1).

Since the SA2 and Scc1 subunits of cohesin can be phosphorylated by P1k1 in vitro (Sumara et al. 2000, Hauf et al. 2005) the inventors want to analyse whether some of the phosphorylation sites detected on Wap1 are dependent on P1k1 in vivo. The inventors treat cells arrested in mitosis by nocodazole with the P1k1 inhibitor BI 2536 for 2 hours or leave them untreated, immunopurified Wap1 and subsequently analyse its phosphorylation state by mass spectrometry like described above. In total 4 phosphorylation sites could never be detected in samples isolated from BI 2536 - treated cells (Figure 1).

These results implicate that Wap1, like the previously described cohesin subunits, is mitotically regulated by phosphorylation and that this regulation is dependent on P1k1 in vivo.

Figure 1: The schematic representation shows the phosphorylation sites detected on Wap1 in mitosis (black), S-phase (white), interphase (grey) and the BI 2536 sensitive (*) mitotic phosphorylation sites.

### Example 2

### Generation of Phospho-Specific Antibodies to Wap1

### a) Generation of Antiserum Against the P1k1 Dependent Phospho-Epitope S465 on Wap1

To generate antibodies that specifically react with a BI 2536 sensitive mitotic phosphorylation site on Wap1, rabbits are immunized with a synthetic phospho-peptide. This peptide represents the part of the Wap1 amino acid sequence which is found by mass spectrometry to be phosphorylated in human cultured cells specifically in mitosis and which is sensitive to BI 2536 (Figure 1).

The peptide is designated 1710 (corresponding to amino acid residues 460 to 471 of human Wap1; SEQ ID NO:1): the carboxy-terminal cysteine residue is added to allow subsequent coupling of the peptides to a carrier protein and is not derived from the Wap1 sequence; the peptide is phosphorylated at serine 465. The antibodies generated are designated 1710 antibodies or short pWap1.

The synthetic peptide is coupled via the carboxy-terminal cysteine residue to keyhole limpet hemocyanin (KLH; Merck Biosciences, Nottingham, UK) following the manufacturer's instructions. 3 mg of lyophilized peptide are dissolved in 700 µl of buffer containing 3 M guanidine hydrochloride and 0.5 M sodium tetraborate, pH 6.9, and the solution is added to 300 µl of the same buffer in which 3 mg KLH has been dissolved. After removal of oxygen by aerating with argon, the reaction mixture is incubated for 1 hour at room temperature and subsequently stored at 4°C. To determine the efficiency of the coupling reaction, 10 µl aliquots of the peptide-KLH solution are removed at the beginning and the end of the reaction, mixed with 50 µl of Ellman's reagent (50 mM sodium acetate, 2 mM 5,5'-Dithiobis(2-nitrobenzoic acid); Sigma-Aldrich, St.Louis, MO), 100 µl solution containing 1 M Tris-HCl, pH 8.0 and 840 µl water, and the absorbance of the samples is measured at 412-nm ("Ellman test"). A decrease in absorbance indicates a reduction in the amount of free thiol groups and is a measure for the efficiency of peptide coupling to KLH (Ellman, 1959; Bulaj et al., 1998). The coupled peptides are used to immunize rabbits at a commercial facility (Gramsch Laboratories, Schwabhausen, Germany). The resulting sera are frozen in 10 ml aliquots in liquid nitrogen and stored at -80 °C.

### b) Generation of Peptide Affinity Columns for the Purification of Phospho-Specific Wap1 Antibodies

To purify phospho-specific antibodies from the rabbit serum, peptide affinity columns are generated. The Wap1 phospho-peptide described above is coupled to a chromatography resin, and the resulting column is then used to bind those antibodies that specifically react with the phospho-peptide. In addition, a peptide is synthesized that is identical in sequences to the sequence of the phospho-peptide, except that it contains an unmodified serine residue instead of the phospho-serine residue. This peptide is also coupled to chromatography resin, and the resulting column is used to remove antibodies from the rabbit sera that react with the Wap1 peptide independently of its phosphorylation state.

To generate the peptide affinity column, Poros beads (Applied Biosystems, Foster City, CA) are activated with the bi-functional cross-linker sulfo-GMBS (Pierce, Rockford, IL) according to the manufacturer's instructions, which result in the generation of maleimid groups. Of the synthetic peptide, 5 mg of lyophilized peptide are dissolved in 3 ml buffer containing 3 M guanidine hydrochloride and 0.1 M Hepes-KOH, pH 7.9, yielding a peptide concentration of ~2.27 µmol. 310 mg of activated Poros beads are mixed with the peptide solution and the suspension is incubated on a rotary shaker for one hour at room temperature. Subsequently, the Poros beads are collected by low speed centrifugation and the coupling reaction is stopped by washing with at least 10 bead volumes of a buffer containing 150 mM NaCl, 20 mM Tris-HCl, pH 7.5. The efficiency of cross-linking is determined by the Ellman test (see a). Resin coupled to peptide is stored in batch at 4°C. To obtain a homogenously packed affinity column the coupled resin is transferred into a HPLC column (0.7 ml; Applied Biosystems) by employing a filling device (Applied Biosystems) that is connected to a BIOCAD HPLC system (Applied Biosystems).

### c) Purification and Concentration of the Phospho-Specific Wap1 Antibodies

Phospho-specific antibodies are purified from the serum generated as described under a) by the following strategy: Antibodies that react with the Wap1 peptide independently of its phosphorylation state are first removed from each serum by passing it over the affinity column that contains the corresponding antigenic peptide in its non-phosphorylated form. Subsequently the serum is passed over a second affinity column which contains the phosphorylated antigenic peptide. Both columns are connected in line to a BIOCAD HPLC and are equilibrated with 20 column volumes (cv) of HBS buffer (20 mM Hepes-KOH, 150 mM NaCl) at a flow rate of 7 ml/min. All chromatography steps are carried out at room temperature and are monitored by measuring the absorbance on-line at 280 nm. Aliquots of antiserum are rapidly thawed in a water bath at 37 °C. The thawed serum is extracted once for two minutes with an equal volume of chloroform to remove lipids. To separate the organic fraction from the serum, the emulsion is centrifuged at 2,000 rpm in a Varifuge 3.0R (Heraeus; Kendro, Asheville, NC) for 15 minutes at 4 °C. Subsequently, the serum is filtered through a 0.45 µm syringe filter (MILLEX-HA; MILLIPORE, Billerica, MA) and stored on ice. Antibodies are purified from 20 ml of serum by four subsequent chromatography runs in each of which 5 ml aliquots are processed. 5 ml of chloroform extracted serum are loaded onto the column with a flow rate of 5 ml/min (column pressure: 20-25 bar). The column is washed with 50 cv of HBS.

Subsequently the two columns are disconnected and bound proteins are eluted separately from the two columns. Bound proteins are eluted first with Buffer A (1.5 M MgCl₂, 100 mM sodium acetate pH 5.2) and subsequently by Buffer B (100 mM glycine-HCl, pH 2.45). The pH of the eluted fractions is immediately neutralized by the addition of 1/10 volume of 2 M Hepes-KOH, pH 7.9. The fractions obtained by elution with Buffer A or with Buffer B are kept separate during all subsequent steps because antibodies eluted by high MgCl₂ concentrations (Buffer A) or by low pH (Buffer B) can differ in their abilities to recognise their antigen under different conditions. The peak fractions are dialyzed over-night at 4 °C against 2 liters of buffer C (20 mM Hepes-KOH, 150 mM NaCl, 10% glycerol), using dialysis tubing with a molecular weight cut-off (MWCO) of 3,500 Da (Spectra/Por; Spectrum Laboratories, Rancho Dominguez, CA). Buffer C is exchanged once after 3 hours. Subsequently, the antibodies are concentrated by ultra-filtration in a Centriprep centrifugal filter device (YM-30, MWCO 30 kDA, 15 ml, Amicon; MILLIPORE) at 3,000 rpm (Varifuge 3.0R) and 4°C. The final protein concentration is determined by photometric measurements at a wavelength of 280 nm. An OD₂₈₀ = 1 of a mixture of IgG and IgM corresponds to 1.4 mg/ml protein (Harlow and Lane, 1988) purity of the samples is assessed by SDS-PAGE and silver staining, and 100 µl aliquots of the purified antibodies are frozen in liquid nitrogen and stored at -80 °C. A typical purification yields 6 to 9 mg of pure antibodies from 20 ml of antiserum.

### d) Characterization of the Specificity of the Phospho-Wap1 Antibodies

The following experiments are performed to determine if the antibodies that are eluted from the affinity column containing phosphorylated antigenic peptide are specifically reacting with a BI 2536 sensitive phosphorylation on Wap1.

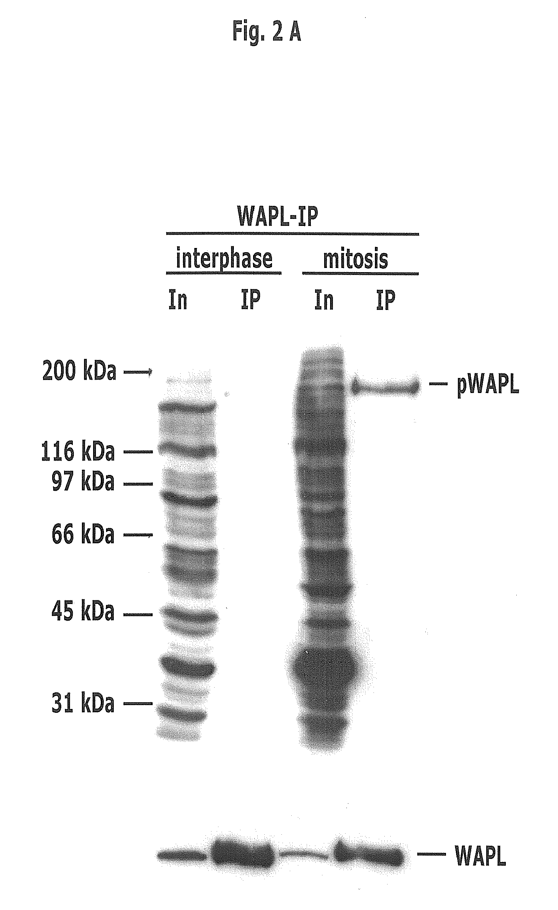

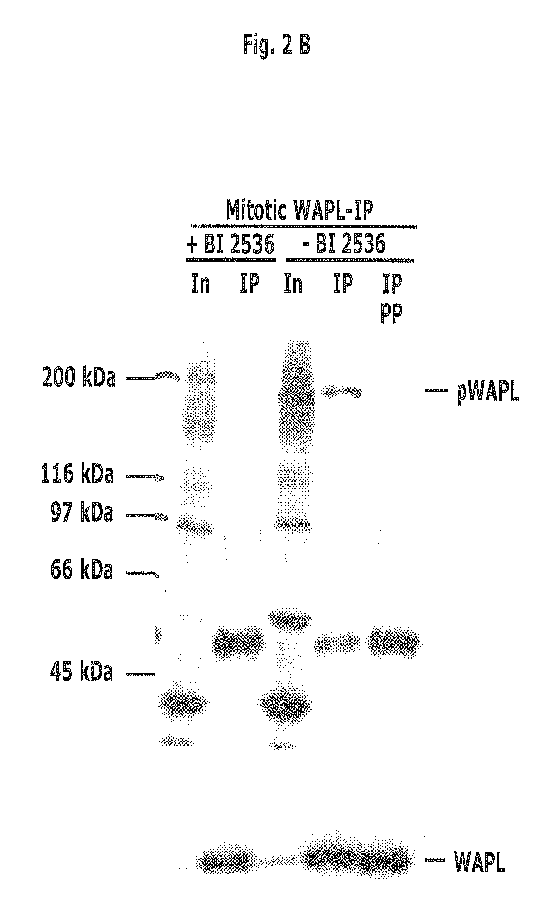

First, Wap1 is isolated by immunoprecipitation with 987 antibodies from cultured human HeLa cells that are either in interphase or arrested in mitosis by treatment with nocodazole. Proteins in the immunoprecipitates as well as the low speed supernatant of the cell extracts are separated by SDS-PAGE and analysed by immunoblotting with the 1710 antibodies. For this purpose, proteins separated by gel electrophoresis are transferred to a PVDF blotting membrane and incubated with the antibodies purified as described under b. The reactivity of the antibodies with proteins bound to the PDVF membrane is detected by incubation with enzyme-linked secondary antibodies that can be detected by enhanced chemoluminescence (ECL). As is shown in Figure 2A, 1710 antibodies only react with Wap1 that has been isolated from mitotic cells where Wap1 is phosphorylated; whereas the antibodies do not react with Wap1 from interphase cells where Wap1 is only phosphorylated on a single residue (see figure 1). There are a number of cross reacting bands detected in the low speed supernatant, indicating that the 1710 antibodies are not mono-specific for Wap1 when probed against a complex protein mixture. To confirm that the 1710 antibodies specifically recognise phosphorylated mitotic Wap1 a fraction of the immunoprecipitates from nocodazole-arrested cells is treated with λ-Phosphatase and analysed by SDS-PAGE and immunoblotting with 1710 antibodies as described above (figure 2B). Phosphatase treatment efficiently abolishes the 1710 signal on mitotic Wap1. These results are consistent with the notion that the reactivity of the antibodies with Wap1 depends on mitotic Wap1 and is phosphorylation specific.

Second, to test whether the phosphorylation site that is detected specifically on mitotic Wap1 is dependent on P1k1 in vivo, Wap1 is immunopurified from nocodazole-arrested cells that are treated or not with the P1k1 inhibitor BI 2536 as described in example 1 and the immunoprecipitates are analysed by SDS-PAGE and western blotting with the 1710 antibodies as described above. Figure 2B clearly shows that 1710 antibodies do not recognise mitotic Wap1 after treatment of mitotic cells with BI 2536. This indicates that the generation of the phosphorylation site detected by the 1710 antibodies is dependent on P1k1 in vivo.

### Figure 2

(A) Low speed supernatants of HeLa cell extracts (In) and immunopurified Wap1 (IP) from either exponentially growing cells or noc arrested cells are separated by SDS-PAGE, transferred to a PVDF membrane, probed with pWap1 and, after stripping, with Wap1 antibodies to control for equal loading.
(B) Low speed supernatants of HeLa cell extracts (In) and immunopurified Wap1 (IP) from noc arrested cells treated or not with BI 2536 are separated by SDS-PAGE, transferred to a PVDF membrane, probed with pWap1 and, after stripping, with Wap1 antibodies to control for equal loading. Immunopurified Wap1 from noc arrested cells is additionally treated with phosphatase (PP).

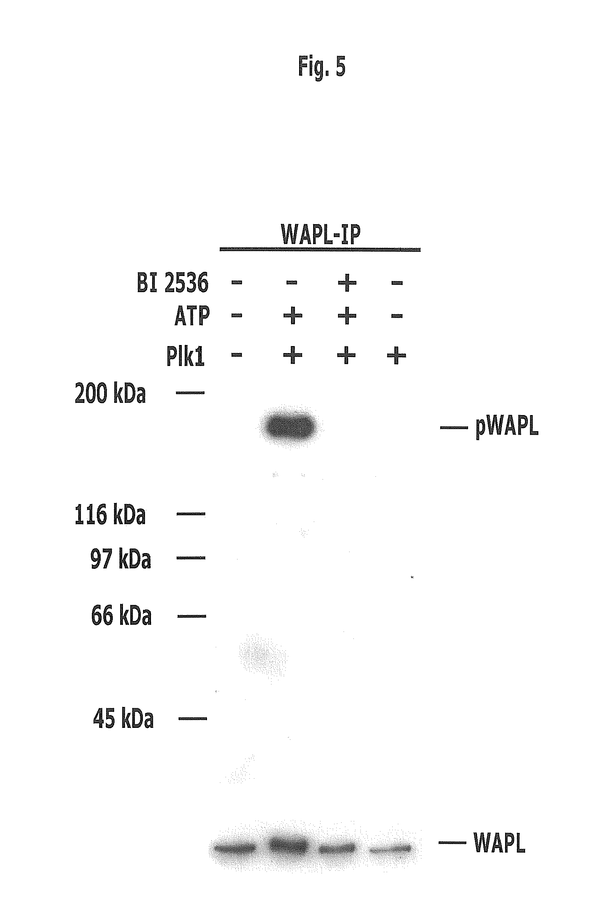

Third, to test whether the reactivity of the 1710 antibodies with Wap1 depends directly on Wap1 phosphorylation by P1k1 the following experiments are performed. Immunoprecipitated interphase Wap1 is incubated in buffer containing MgCl₂ and recombinant active 6xHis-tagged-P1k1 enzyme supplemented or not with ATP and BI 2536. Subsequently, Wap1 is analysed by SDS-PAGE and immunoblotting with the 1710 antibodies as described above. As is shown in Figure 5 the pre-treatment of interphase Wap1 with MgCl₂, ATP and P1k1 allows the antibodies to react with Wap1, whereas interphase Wap1 pre-incubated with MgCl₂, P1k1 ATP and BI 2536 is not recognised. Also, interphase Wap1 pre-incubated with MgCl₂ and P1k1 in the absence of ATP is not recognised by 1710. These data imply that the reactivity of the antibodies with Wap1 directly depends on the prior phosphorylation of Wap1 by P1k1 in vitro.

### Figure 5

Wap1 is immunopurified by 987 antibodies from extracts of exponentially growing HeLa cells and in vitro phosphorylated with recombinant P1k1 in presence or absence of either BI 2536 or ATP. S indicates immunopurified Wap1 before the kinase assay.

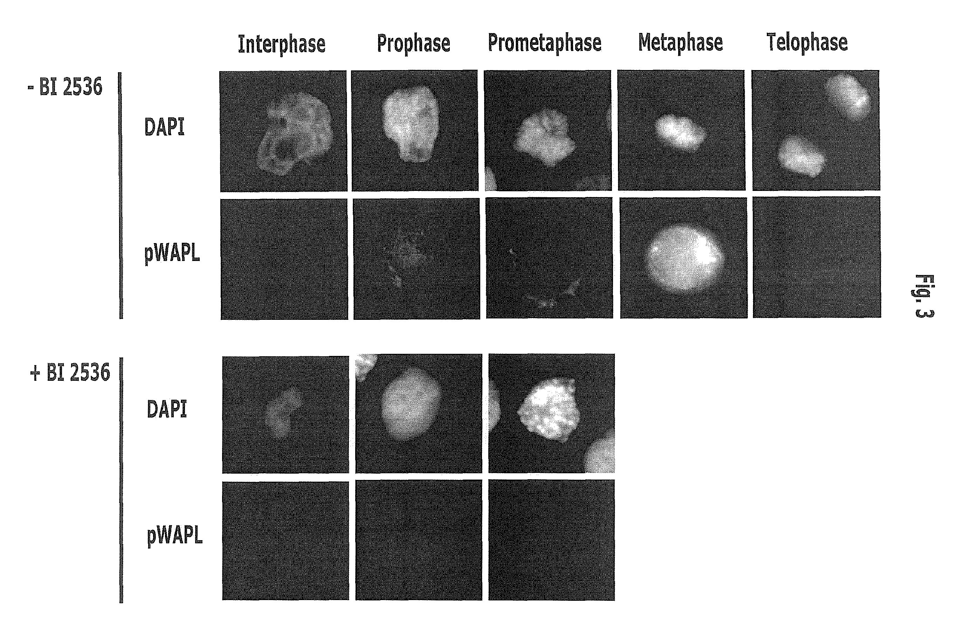

Fourth, to test whether the 1710 antibodies can also specifically recognize mitotic cells in immunocytological stainings, the following experiments are performed. HeLa cells are grown on cover slips, treated with BI 2536 for 45 minutes or not, fixed with paraformaldehyde and stained with the 1710 antibodies as well as with DAPI and analysed by immunofluorescence microscopy. Representative images of stained cells in interphase and the mitotic phases prophase, prometaphase, metaphase and telophase are shown in figure 3:
HeLa Kyoto cells are grown on cover slips, treated or not with 64 nM BI 2536, fixed and stained with DAPI and pWap1. Representative cells from each cell cycle state are shown.

The 1710 antibodies stain cells from prophase to metaphase but not cells in telophase nor cells in interphase. The 1710 signal localizes to the nucleus in prophase but can then be detected throughout the cells after nuclear envelope breakdown in prometaphase until metaphase. The cells that have been treated with BI 2536 prior to fixation, staining and analysis do not show any staining with the 1710 antibodies in any of the cell cycle phases. Since cells treated with BI 2536 arrest at prometaphase, cells in metaphase and telophase are not present in this experiment. These data confirm that the 1710 antibodies recognise a BI 2536 dependent mitotic signal.

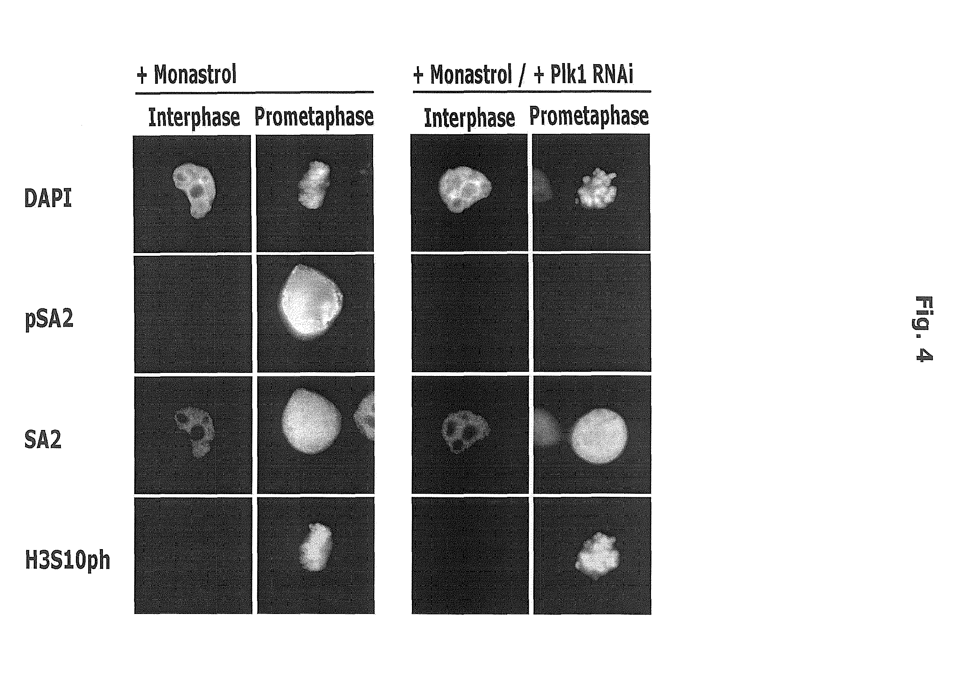

To find out whether the 1710 antibodies only recognise phoshporylated Wap1 in fixed cells, the staining pattern of 1710 is compared with the known Wap1 localization (S. Kueng, B. Hegemann, B. Peters, J.J. Lipp, A. Schleiffer, K. Mechtler and J.-M. Peters, unpublished observations). It is noted that the 1710 staining does not clearly resemble the Wap1 staining. To test whether the 1710 signal in fixed cells depends on Wap1, Wap1 is depleted from cells by siRNA. Cells are then fixed, stained and analysed by immunofluorescence microscopy as described above. The 1710 antibodies signal does not decrease in response to Wap1 depletion (data not shown), indicating that the 1710 antibodies recognise an unknown antigen but not Wap1 in fixed cells.
To confirm that the effect of BI 2536 on the 1710 signal in fixed mitotic cells really depends on P1k1, P1k1 is depleted by a siRNA oligonucleotide specific to P1k1 prior to fixation and staining with 1710 antibodies, DAPI, antibodies that recognise SA2 independent of its phosphorylation state (anti-SA2, Bethyl), anti histone H3-phospho-Ser10 (H3S10ph) antibodies (Cell Signalling). Stained cells are analysed as described above. Cells depleted of P1k1 arrest at prometaphase (Sumara et al. 2004) so meta- and telophase cells are not present in the RNAi experiment. Just like in BI 2536- treated cells, the 1710 signal is completely abolished in P1k1 siRNA- treated cells from all cell cycle phases (figure 4). These results confirm that the generation of the antigen that the 1710 antibodies recognises in fixed mitotic cells is dependent on P1k1.

### Figure 4

HeLa Kyoto cells are grown on cover slips, treated or not with P1k1 siRNA oligos, treated with 0.1 mM monastrol for five hours, fixed and stained with DAPI, pWap1, SA2 and H3S10ph. Representative interphase and prometaphase arrest cells are shown.

### Example 3

A combination of chemical biology and mass spectrometry to detect P1k1 dependent phosphorylation sites on Wap1.

From previous studies (Sumara et al. 2002, Hauf et al. 2005) it is known that the Cohesin subunits Scc1 and SA2 are phosphorylated in mitosis in vitro by P1k1. To test if Wap1, a loosely associated cohesin subunits, is also phosphorylated by P1k1 in vivo, Wap1 is analysed in an experiment combining chemical biology and mass spectrometry as describe below.

HeLa cells are grown to 90% confluency and arrested in mitosis by nocodazole for a total of 16 hours. The P1k1 inhibitor BI 2536 is added 14 hours after nocodazole for a total of two hours. Control cells are left untreated. Mitotic cells are then harvested by mitotic shake off to separate them from interphase cells. To remove cell culture medium, cells are washed two times with ice cold PBS, frozen in liquid nitrogen and then stored at - 80°C. To extract soluble proteins from the cell pellets, they are resuspended in lysis buffer (buffer A) containing 20 mM Tris-HCl, pH 7.5; 150 mM NaCl, 5 mM EDTA, 20 mM beta-glycerophosphate, 10 mM NaF, 10 % glycerol, 0.1 % NP-40, 1 µM Okadaic acid, 0.2 mM NaVO4, 1 mM DTT and a protease inhibitor mix (Chymostatin, Leupeptin and Pepstatin at 10 µg / ml). Cells are lysed by extraction in a glass homogeniser for 10 minutes. Unsoluble proteins are removed by spinning twice at 13,000 rpm for 15 minutes. The supernatant is added to Affiprep beads (Amersham) containing cross-linked 987 antibodies and rotated overhead at 4°C for 1 hour followed by 6 extensive washes in buffer A and 5 washes in TBS to remove detergent. Proteins are eluted by adding 1.5x bead volumes of 0.2 M glycine pH 2.0. Eluates are adjusted to pH 8 by addition of 1.5 M Tris pH 9.2, subjected to reduction by 0.5 mg DTT / ml for 30 minutes at 56°C and alkylation by 2.5 mg / ml Iodoacetamide for 30 minutes. Samples are then digested in parallel with either 50 ng / ml of trypsin, chymotrypsin or GluC endoproteases for 16 hours, 4 hours or 16 hours respectively. Each digest is analysed by LC-ESI-MS/MS followed by spectra evaluation as described in materials and methods.

Detected phosphopeptides from the nocodazole - arrested sample and the nocodazole - arrested sample treated with BI 2536 are compared. Phosphorylated peptides which are present in both samples are considered to be P1k1 independent. Phosphorylated peptides which are not present in the BI 2536 - treated sample but are present in the control sample are considered P1k1 dependent only if the corresponding unphosphorylated peptide was detected in the BI 2536 - treated sample. In the analysed samples, the detected peptides cover 87% and 86% of the Wap1 sequence, in the untreated or treated sample respectively. The analysis shows that the phosphorylation sites on serine 465, on serine 528, on serine 988 and on serine 1154 are dependent on P1k1.

### References

Barr FA, Sillje HH, Nigg EA (2004) Polo-like kinases and the orchestration of cell division. Nat Rev Mol Cell Biol 5(6): 429-440.
Beck HC, Nielsen EC, Matthiesen R, Jensen LH, Sehested M, Finn P, Grauslund M, Hansen AM, Jensen ON (2006).Mol Cell Proteomics 1314-25
Bulaj, G., Kortemme, T. and Goldenberg, D. P. (1998). Ionization-reactivity relationships for cysteine thiols in polypeptides. Biochemistry 37, 8965-8972.
Cantin GT, Yates JR 3rd (2004). J Chromatogr A. 1053(1-2):7-14.
Cyert, M.S., T. Scherson, and M.W. Kirschner (1988). Monoclonal antibodies specific for thiophosphorylated proteins recognize Xenopus MPF. Dev Biol 129: 209-216
Davis FM, Tsao TY, Fowler SK, Rao PN. Monoclonal antibodies to mitotic cells (1983).Proc Natl Acad Sci U S A (10): 2926-30.
Ditchfield C., Johnson VL., Tighe A., Ellston R., Haworth C., Johnson T., Mortlock A., Keen N., Taylor SS. (2003). Aurora B couples chromosome alignment with anaphase by targeting BubR1, Mad2, and Cenp-E to kinetochores. J Cell Biol. Arp. 28, 161(2):267-80.
Druker and Lydon, J Clin Invest. (2000) Jan;105(1):3-7.
Ellman GL. (1959), Tissue sulfhydryl groups. Arch Biochem Biophys., May, 82(1):70-7.
Fry, D.W., Bedford, D.C., Harvey, P.H., Fritsch, A., Keller, PR., Wu, Z., Dobrusin, E., Leopold, W.R., Fattaey (2001) J Biol Chem 276, 16617-16623.
Garin-Chesa, P., Old LJ., Rettig WJ., (1990), Cell surface glycoprotein of reactive stromal fibroblasts as a potential antibody target in human epithelial cancers. Proc Natl Acad Scie USA Sep 87(18):7235-9.
Giménez-Abian JF, Sumara I, Hirota T, Hauf S, Gerlich D et al. (2004). Regulation of Sister Chromatid Cohesion between Chromosome Arms. Curr Biol. 2004 Jul 13; 14(13):1187-93.
Graves, P.R., Yu, L., Schwarz, J.K., Gales, J., Sausville, E.A., O'Connor, P.M., Piwnica-Worms, H. (2000). J. Biol. Chem 275, 5600-5605.
Greiner D, Bonaldi T, Eskeland R, Roemer E, Imhof A (2005). Nat Chem Biol 143-5
Harlow, E. and Lane, D. (Eds.), (1988). Antibodies. A laboratory manual. Cold Spring Harbor laboratory Press. N.Y.
Hauf S, Cole RW, LaTerra S, Zimmer C, Schnapp G et al. (2003). The small molecule Hesperadin reveals a role for Aurora B in correcting kinetochore-microtubule attachment and in maintaining the spindle assembly checkpoint. J Cell Biol 161(2): 281-294.
Hauf S., Roitinger E., Koch B., Dittrich CM., Mechtler K., Peters JM. (2005). Dissociation of cohesin from chromosome arms and loss of arm cohesion during early mitosis depends on phosphorylation of SA2. PLoS Biol., Mar. 3(3):e69.
Hirota T, Gerlich D, Koch B, Ellenberg J, Peters JM (2004).J Cell Sci. 117(Pt 26):6435-45
Kennet, (1980)."Monoclonal Antibodies, Hybridomas--A New Dimension in Biological Analysis", Eds. Kennet, McKern and Bechtol, Plenum Press, N.Y (1980).
Kitada et al., Mol. Cell. Biol. 1993, 13: 4445-4457;
Kohler and Milstein, (1975), Nature 256: 495.
Kraft C, Herzog F, Gieffers C, Mechtler K, Hagting A et al. (2003). Mitotic regulation of the human anaphase-promoting complex by phosphorylation. Embo J 22(24): 6598-6609.
Lane and Nigg, J. Cell Biol. 1996, 135: 1701-1713.
Lewis at al. (1994). THE JOURNAL OF BIOLOGICAL CHEMISTRY, Vol. 269, No. 42, Issue of October 21, pp. 26259-26266.
Llamazares et al., Genes Dev. 1991, 5: 2153-2165.
Liu and Erikson, PNAS 2002, 99: 8672-8676.
Liu and Erikson, PNAS 2003, 100: 5789-5794.
Losada A, Hirano M, Hirano T (2002). Cohesin release is required for sister chromatid resolution, but not for condensin-mediated compaction, at the onset of mitosis. Genes Dev 16(23): 3004-3016.
Meijer L, Borgne A, Mulner O, Chong JP, Blow JJ, Inagaki N, Inagaki M, Delcros JG, Moulinoux JP (1997).Eur J Biochem. 243(1-2):527-36.
Minucci S, Horn V, Bhattacharyya N, Russanova V, Ogryzko VV, Gabriele L, Howard BH, Ozato K (1997). Proc Natl Acad Sci U S A 94(21):11295-300.
Naiem M, Gerdes J, Abdulaziz Z, Stein H, Mason DY (1983). Production of a monoclonal antibody reactive with human dendritic reticulum cells and its use in the immunohistological analysis of lymphoid tissue. J Clin Pathol. 36(2):167-75.
Nasmyth K., Peters JM., Uhlmann F., (2001). Splitting the chromosome: cutting the ties that bind sister chromatids. Novartis Found Symp. 237:113-33
Nawa G, Ueda T, Mori S, Yoshikawa H, Fukuda H, Ishiguro S, Funai H, Uchida A (1996). Int J Cancer 69(2):86-91.
Rice JC, Briggs SD, Ueberheide B, Barber CM, Shabanowitz J, Hunt DF, Shinkai Y, Allis CD (2003).Mol Cell 12(6):1591-8.
Rena et al. (1999). J Biol Chem, June 11, Vol. 274, Issue 24, 17179-17183. Schluter C, Duchrow M, Wohlenberg C, Becker MH, Key G, Flad HD, Gerdes J (1993). The cell proliferation-associated antigen of antibody Ki-67: a very large, ubiquitous nuclear protein with numerous repeated elements, representing a new kind of cell cycle-maintaining proteins. J Cell Biol 123(3):513-22.
Spänkuch-Schmitt et al., Oncogene 2002, 21: 3162-3171;
Strebhardt K., Ullrich A. (2006). Targeting polo-like kinase 1 for cancer therapy. Nat Rev Cancer, Apr. 6(4):321-30.
Sumara I, Vorlaufer E, Gieffers C, Peters BH, Peters JM (2000). Characterization of vertebrate cohesin complexes and their regulation in prophase. J Cell Biol 151(4): 749-762.
Sumara I, Vorlaufer E, Stukenberg PT, Kelm O, Redemann N et al. (2002). The dissociation of cohesin from chromosomes in prophase is regulated by Polo-like kinase. Mol Cell 9(3): 515-525.
Sumara et al., Curr. Biol. 2004, 14: 1712-1722.
van Vugt MA., van de Weerdt BC., Vader G., Janssen H., Calafat J., Klompmaker R., Wolthuis RM., Medema RH. (2004). Polo-like kinsase-1 is required for bipolar spindle formation but is dispensable for anaphase promoting complex/Cdc20 activation and initiation of cytokineses. J Biol Chem. Aug. 27; 279(35):36841-54.
Waizenegger IC, Hauf S, Meinke A, Peters JM (2000). Two distinct pathways remove mammalian cohesin from chromosome arms in prophase and from centromeres in anaphase. Cell 103(3): 399-410.
Wang, Y, Li, J., Booher, R.N., Kraker, A., Lawrence, T., Leopold, W.R. and Sun, Y (2001). Cancer Res 61, 8211-8217.
Westendorf JM, Rao PN, Gerace L (1994). Cloning of cDNAs for M-phase phosphoproteins recognized by the MPM2 monoclonal antibody and determination of the phosphorylated epitope. Proc Natl Acad Sci U S A 91(2): 714-8.
Wisdom, G.B., (1994), Peptide Antigens, A Practical Approach, Oxford University Press, Eds. Rickwood D. And Hames B.D.
Yates JR, 3rd, Link AJ, Schieltz D (2000). Direct analysis of proteins in mixtures. Application to protein complexes. Methods Mol Biol 146: 17-26.
Yuan, J., Horlin, A., Hock, B., Stutte, HJ., Rubsamen-Waigmann, H., and Strebhardt, K. (1997). American Journal of Pathology, Apr;150(4):1165-72.

## Claims

1. An antibody that binds to a phosphorylation site of the human cohesin subunit Wap1 (SEQ ID NO:1) when said phosphorylation site of Wap1 is phosphorylated by P1k1 and that does not bind to said potential phosphorylation site when said phosphorylation site is not phosphorylated.

2. The antibody of claim 1 that does not bind to said potential phosphorylation site when said phosphorylation by P1k1 is inhibited.

3. The antibody of claim 1 or 2, which binds to
i. one or more P1k1-dependent phosphorylations at serine 465, serine 528, serine 988 or at serine 1154 of Wap1 (SEQ ID NO: 1) and/or to
ii. one or more P1k1-dependent phosphorylations on another P1k1 substrate, wherein said phosphorylations are recognized by an antibody generated against a phosphorylation site of Wap1 defined in i).

4. A method for detecting P1k1 activity in a cell *ex vivo* or *in vitro,* wherein the presence of a phosphorylated P1k1 substrate is determined by
a. contacting a biological sample suspected of P1k1 activity with one or more phospho-specific antibodies of any ones of claims 1 to 3 and
b. determining binding of said antibody or antibodies to phosphorylated Wap1 or said other P1k1 substrate, wherein the amount of bound antibody is indicative of the level of P1k1 activity.

5. A method for determining a compound's effect on P1k1 activity in an individual treated with said compound by determining *ex vivo,* before and after administration of said compound to said individual, the presence of P1k1-dependent phosphorylation in a sample, wherein
a) a sample obtained from said individual is contacted with one or more antibodies of any ones of claims 1 to 3,
b) binding of said antibody or antibodies to Wap1 or said other P1k1 substrate is determined, wherein the amount of bound antibody or antibodies is indicative of the level of P1k1 activity,
c) the levels of phosphorylated Wap1 or of said other P1k1 substrate, respectively, before and after administration of said compound are compared,
wherein a reduced level of phosphorylated P1k1 substrate upon administration of said compound is indicative of its inhibitory effect on P1k1.

6. The method of claim 4, wherein said compound's effect on P1k1 activity is determined over a period of time at pre-defined time points by comparing the levels of phosphorylated P1k1 substrate over said period of time,
wherein a reduced level of phosphorylated P1k1 substrate is indicative of said compound's effect on P1k1 activity.

7. A method for selecting a cancer patient for being treated with a compound that acts as inhibitor of P1k1, by determining *ex vivo,* the presence of phosphorylated P1k1 substrate in cells of said patient, wherein
a) a sample obtained from a tumor of said patient is contacted with one or more antibodies of any one of claims 1 to 3,
b) binding of said antibody or antibodies to Wap1 or to said other P1k1 substrate is determined,
wherein a high level phosphorylation of Wap1 or to said other P1k1 substrate is indicative of aberrant P1k1 activity and a patient exhibiting such high phosphorylation level is selected for treatment with said P1k1 inhibitory compound.

8. The use of an antibody of any ones of claims 1 to 3 in a method for determining or confirming a compound's ability to inhibit P1k1 in a cell,
wherein
a) a population of cells having P1k1 activity is adhered to a first solid support and contacted with a candidate compound for a period of time sufficient for the compound to exert its inhibitory effect on P1k1,
b) a capture antibody that binds to Wap1 or to said other P1k1 substrate at a site different from a P1k1-dependent phosphorylation site is adhered to a second solid support,
c) a lysate of cells obtained from step a. is added to said second solid support to capture Wap1 or to said other P1k1 substrate respectively,
d) unbound cell lysate is removed from the second solid support
e) the captured P1k1 substrate is exposed to a antibody of claims 1 or 2 that carries a detectable label,
f) binding of said labeled antibody to the captured Wap1 or P1k1 substrate is measured,
g) the signal obtained in step f. is compared with the signal obtained from control cells obtained without exposure of compound, wherein a reduction of the signal as compared to the control cells is indicative of said compound's inhibitory effect on P1k1.

9. A method for treating a patient or a patient population having a tumor that correlates with P1k1 acitivity, comprising the steps of
a) determining whether the tumor of said patient contains one or more P1k1-dependent phosphorylations,
b) selecting a patient or patient population whose tumor has one or more P1k1-dependent phosphorylation(s),
c) administering to said patient a therapeutically effective amount of a P1k1 inhibitor.

10. The method of claim 9, wherein step b) comprises determining one or more P1k1-dependent phosphorylations on Wap1 or another P1k1 substrate,
wherein said phosphorylations are recognized by an antibody generated against a phosphorylation site of Wap1.

11. The method of claim 9, wherein step b) comprises determining one or more P1k1-dependent phosphorylations
i. at serine 465, serine 528, serine 988 or at serine 1154 on Wap1 (SEQ ID NO: 1) and/or
ii. on another P1k1 substrate, wherein said phosphorylations are recognized by an antibody generated against a phosphorylation site of Wap1 defined in i).

12. The method of claim 9, wherein said P1k1 inhibitor administered in step c) is a compound of the formula


SEQUENCE LISTING

<110>  Boehringer Ingelheim International GmbH

<120>  Biomarkers for Polo-like kinase 1 activity

<130>  Case 14-0088

<160>  1

<170>  PatentIn version 3.3

<210>  1

<211>  1275

<212>  PRT

<213>  Homo sapiens

<400>  2

Met Val Gln Gly Pro Val Gln Thr Pro Ala Leu Thr Ile His Arg Arg
1               5                   10                  15

Lys Arg Arg Arg Ser Cys Pro Pro Asn Arg Ala Ser Tyr Leu Pro Lys
            20                  25                  30

Ala Glu Ser Leu Ala Ser Leu Gly Ser His Leu Pro Ala Leu Leu Ser
        35                  40                  45

Arg Ala Arg Val Pro Arg Pro Pro Ala Gly Arg Arg Glu Arg Glu Arg
    50                  55                  60

Arg Arg Arg Pro Val Ala Lys Ala Pro Ala Arg Leu Arg Gly Glu Tyr
65                  70                  75                  80

Glu Thr Gly Val Lys Met Thr Ser Arg Phe Gly Lys Thr Tyr Ser Arg
                85                  90                  95

Lys Gly Gly Asn Gly Ser Ser Lys Phe Asp Glu Val Phe Ser Asn Lys
            100                 105                 110

Arg Thr Thr Leu Ser Thr Lys Trp Gly Glu Thr Thr Phe Met Ala Lys
        115                 120                 125

Leu Gly Gln Lys Arg Pro Asn Phe Lys Pro Asp Ile Gln Glu Ile Pro
    130                 135                 140

Lys Lys Pro Lys Val Glu Glu Glu Ser Thr Gly Asp Pro Phe Gly Phe
145                 150                 155                 160

Asp Ser Asp Asp Glu Ser Leu Pro Val Ser Ser Lys Asn Leu Ala Gln
                165                 170                 175

Val Lys Cys Ser Ser Tyr Ser Glu Ser Ser Glu Ala Ala Gln Leu Glu
            180                 185                 190

Glu Val Thr Ser Val Leu Glu Ala Asn Ser Lys Ile Ser His Val Val
        195                 200                 205

Val Glu Asp Thr Val Val Ser Asp Lys Cys Phe Pro Leu Glu Asp Thr
    210                 215                 220

Leu Leu Gly Lys Glu Lys Ser Thr Asn Arg Ile Val Glu Asp Asp Ala
225                 230                 235                 240

Ser Ile Ser Ser Cys Asn Lys Leu Ile Thr Ser Asp Lys Val Glu Asn
                245                 250                 255

Phe His Glu Glu His Glu Lys Asn Ser His His Ile His Lys Asn Ala
            260                 265                 270

Asp Asp Ser Thr Lys Lys Pro Asn Ala Glu Thr Thr Val Ala Ser Glu
        275                 280                 285

Ile Lys Glu Thr Asn Asp Thr Trp Asn Ser Gln Phe Gly Lys Arg Pro
    290                 295                 300

Glu Ser Pro Ser Glu Ile Ser Pro Ile Lys Gly Ser Val Arg Thr Gly
305                 310                 315                 320

Leu Phe Glu Trp Asp Asn Asp Phe Glu Asp Ile Arg Ser Glu Asp Cys
                325                 330                 335

Ile Leu Ser Leu Asp Ser Asp Pro Leu Leu Glu Met Lys Asp Asp Asp
            340                 345                 350

Phe Lys Asn Arg Leu Glu Asn Leu Asn Glu Ala Ile Glu Glu Asp Ile
        355                 360                 365

Val Gln Ser Val Leu Arg Pro Thr Asn Cys Arg Thr Tyr Cys Arg Ala
    370                 375                 380

Asn Lys Thr Lys Ser Ser Gln Gly Ala Ser Asn Phe Asp Lys Leu Met
385                 390                 395                 400

Asp Gly Thr Ser Gln Ala Leu Ala Lys Ala Asn Ser Glu Ser Ser Lys
                405                 410                 415

Asp Gly Leu Asn Gln Ala Lys Lys Gly Gly Val Ser Cys Gly Thr Ser
            420                 425                 430

Phe Arg Gly Thr Val Gly Arg Thr Arg Asp Tyr Thr Val Leu His Pro
        435                 440                 445

Ser Cys Leu Ser Val Cys Asn Val Thr Ile Gln Asp Thr Met Glu Arg
    450                 455                 460

Ser Met Asp Glu Phe Thr Ala Ser Thr Pro Ala Asp Leu Gly Glu Ala
465                 470                 475                 480

Gly Arg Leu Arg Lys Lys Ala Asp Ile Ala Thr Ser Lys Thr Thr Thr
                485                 490                 495

Arg Phe Arg Pro Ser Asn Thr Lys Ser Lys Lys Asp Val Lys Leu Glu
            500                 505                 510

Phe Phe Gly Phe Glu Asp His Glu Thr Gly Gly Asp Glu Gly Gly Ser
        515                 520                 525

Gly Ser Ser Asn Tyr Lys Ile Lys Tyr Phe Gly Phe Asp Asp Leu Ser
    530                 535                 540

Glu Ser Glu Asp Asp Glu Asp Asp Asp Cys Gln Val Glu Arg Lys Thr
545                 550                 555                 560

Ser Lys Lys Arg Thr Lys Thr Ala Pro Ser Pro Ser Leu Gln Pro Pro
                565                 570                 575

Pro Glu Ser Asn Asp Asn Ser Gln Asp Ser Gln Ser Gly Thr Asn Asn
            580                 585                 590

Ala Glu Asn Leu Asp Phe Thr Glu Asp Leu Pro Gly Val Pro Glu Ser
        595                 600                 605

Val Lys Lys Pro Ile Asn Lys Gln Gly Asp Lys Ser Lys Glu Asn Thr
    610                 615                 620

Arg Lys Ile Phe Ser Gly Pro Lys Arg Ser Pro Thr Lys Ala Val Tyr
625                 630                 635                 640

Asn Ala Arg His Trp Asn His Pro Asp Ser Glu Glu Leu Pro Gly Pro
                645                 650                 655

Pro Val Val Lys Pro Gln Ser Val Thr Val Arg Leu Ser Ser Lys Glu
            660                 665                 670

Pro Asn Gln Lys Asp Asp Gly Val Phe Lys Ala Pro Ala Pro Pro Ser
        675                 680                 685

Lys Val Ile Lys Thr Val Thr Ile Pro Thr Gln Pro Tyr Gln Asp Ile
    690                 695                 700

Val Thr Ala Leu Lys Cys Arg Arg Glu Asp Lys Glu Leu Tyr Thr Val
705                 710                 715                 720

Val Gln His Val Lys His Phe Asn Asp Val Val Glu Phe Gly Glu Asn
                725                 730                 735

Gln Glu Phe Thr Asp Asp Ile Glu Tyr Leu Leu Ser Gly Leu Lys Ser
            740                 745                 750

Thr Gln Pro Leu Asn Thr Arg Cys Leu Ser Val Ile Ser Leu Ala Thr
        755                 760                 765

Lys Cys Ala Met Pro Ser Phe Arg Met His Leu Arg Ala His Gly Met
    770                 775                 780

Val Ala Met Val Phe Lys Thr Leu Asp Asp Ser Gln His His Gln Asn
785                 790                 795                 800

Leu Ser Leu Cys Thr Ala Ala Leu Met Tyr Ile Leu Ser Arg Asp Arg
                805                 810                 815

Leu Asn Met Asp Leu Asp Arg Ala Ser Leu Asp Leu Met Ile Arg Leu
            820                 825                 830

Leu Glu Leu Glu Gln Asp Ala Ser Ser Ala Lys Leu Leu Asn Glu Lys
        835                 840                 845

Asp Met Asn Lys Ile Lys Glu Lys Ile Arg Arg Leu Cys Glu Thr Val
    850                 855                 860

His Asn Lys His Leu Asp Leu Glu Asn Ile Thr Thr Gly His Leu Ala
865                 870                 875                 880

Met Glu Thr Leu Leu Ser Leu Thr Ser Lys Arg Ala Gly Asp Trp Phe
                885                 890                 895

Lys Glu Glu Leu Arg Leu Leu Gly Gly Leu Asp His Ile Val Asp Lys
            900                 905                 910

Val Lys Glu Cys Val Asp His Leu Ser Arg Asp Glu Asp Glu Glu Lys
        915                 920                 925

Leu Val Ala Ser Leu Trp Gly Ala Glu Arg Cys Leu Arg Val Leu Glu
    930                 935                 940

Ser Val Thr Val His Asn Pro Glu Asn Gln Ser Tyr Leu Ile Ala Tyr
945                 950                 955                 960

Lys Asp Ser Gln Leu Ile Val Ser Ser Ala Lys Ala Leu Gln His Cys
                965                 970                 975

Glu Glu Leu Ile Gln Gln Tyr Asn Arg Ala Glu Asp Ser Ile Cys Leu
            980                 985                 990

Ala Asp Ser Lys Pro Leu Pro His Gln Asn Val Thr Asn His Val Gly
        995                 1000                1005

Lys Ala Val Glu Asp Cys Met Arg Ala Ile Ile Gly Val Leu Leu
    1010                1015                1020

Asn Leu  Thr Asn Asp Asn Glu  Trp Gly Ser Thr Lys  Thr Gly Glu
    1025                1030                1035

Gln Asp  Gly Leu Ile Gly Thr  Ala Leu Asn Cys Val  Leu Gln Val
    1040                1045                1050

Pro Lys  Tyr Leu Pro Gln Glu  Gln Arg Phe Asp Ile  Arg Val Leu
    1055                1060                1065

Gly Leu  Gly Leu Leu Ile Asn  Leu Val Glu Tyr Ser  Ala Arg Asn
    1070                1075                1080

Arg His  Cys Leu Val Asn Met  Glu Thr Ser Cys Ser  Phe Asp Ser
    1085                1090                1095

Ser Ile  Cys Ser Gly Glu Gly  Asp Asp Ser Leu Arg  Ile Gly Gly
    1100                1105                1110

Gln Val  His Ala Val Gln Ala  Leu Val Gln Leu Phe  Leu Glu Arg
    1115                1120                1125

Glu Arg  Ala Ala Gln Leu Ala  Glu Ser Lys Thr Asp  Glu Leu Ile
    1130                1135                1140

Lys Asp  Ala Pro Thr Thr Gln  His Asp Lys Ser Gly  Glu Trp Gln
    1145                1150                1155

Glu Thr  Ser Gly Glu Ile Gln  Trp Val Ser Thr Glu  Lys Thr Asp
    1160                1165                1170

Gly Thr  Glu Glu Lys His Lys  Lys Glu Glu Glu Asp  Glu Glu Leu
    1175                1180                1185

Asp Leu  Asn Lys Ala Leu Gln  His Ala Gly Lys His  Met Glu Asp
    1190                1195                1200

Cys Ile  Val Ala Ser Tyr Thr  Ala Leu Leu Leu Gly  Cys Leu Cys
    1205                1210                1215

Gln Glu  Ser Pro Ile Asn Val  Thr Thr Val Arg Glu  Tyr Leu Pro
    1220                1225                1230

Glu Gly  Asp Phe Ser Ile Met  Thr Glu Met Leu Lys  Lys Phe Leu
    1235                1240                1245

Ser Phe  Met Asn Leu Thr Cys  Ala Val Gly Thr Thr  Gly Gln Lys
    1250                1255                1260

Ser Ile  Ser Arg Val Ile Glu  Tyr Leu Glu His Cys
    1265                1270                1275